# EUROPEAN PATENT APPLICATION

(11) **EP 2 446 823 A1**
(43) Date of publication of application: **02.05.2012**
(21) Application number: 11178976.4
(22) Date of filing: 26.08.2011
(51) Int. Cl.: A61B 8/00, A61B 8/08, A61N 7/00

(54) **Ultrasonic diagnostic apparatus**

(30) Priority: 02.11.2010 KR 20100108217
(71) Applicant: Samsung Medison Co., Ltd., Nam-myun Hongchun-gun Kangwon do 250-875 (KR)
(72) Inventor: Ryu, Yeon, Namyangju-si, Gyeonggi-do (KR)
(74) Representative: Schmid, Wolfgang

(57) **Abstract**

Disclosed herein is an ultrasonic diagnostic apparatus. The apparatus transmits ultrasonic signals to an object and receives reflected ultrasonic signals from the object to create ultrasonic images. The apparatus includes a detection unit (130) that detects an ultrasonic influence factor indicative of the influence of ultrasonic waves upon biological tissues, a warning unit (140) that warns a user, and a controller (110) that receives the ultrasonic influence factor from the detection unit (130) and activates the warning unit if the ultrasonic influence factor exceeds a predetermined threshold value.

## Description

### TECHNICAL FIELD

The present invention relates to an ultrasonic diagnostic apparatus which, during ultrasonic diagnosis, detects an ultrasonic influence factor that can affect a target body, and gives a warning to a user based on the detected results, thereby performing ultrasonic diagnosis safe for the body.

### BACKGROUND

Ultrasonic diagnostic apparatuses are used in a wide range of applications. For example, ultrasonic diagnostic apparatuses are extensively used in medicine due to non-invasive and non-destructive characteristics thereof. Recently, high performance ultrasonic diagnostic apparatuses have been used to provide two-dimensional or three-dimensional images of internal organs.

Generally, ultrasonic diagnostic apparatuses electrically stimulate a transducer to generate ultrasonic signals, and transmit the ultrasonic signals to an object like the body of a patient. The ultrasonic signals transmitted to the object are reflected at boundaries between discontinuous tissues in the object, and the reflected ultrasonic signals are transmitted to a transducer and converted into electric signals. The converted electric signals are processed to create ultrasonic images of the object.

For medical ultrasonic diagnostic apparatuses, which are used in medical institutions for diagnosing the organs of the body, particularly, position, size, motion, heartbeat, etc. of an unborn baby or the like, it is known that, from a medical point of view, the apparatuses are safe and harmless to an unborn baby. However, even though there is no evidence of harm to an unborn baby, it may be difficult to guarantee that ultrasonic scanning is completely harmless to an unborn baby or the like for long term, because ultrasonic waves may cause physical influence upon or temperature increase in biological tissues.

Thus, it is needed for an operator or diagnostician to pay attention such that the target body such as an unborn baby is not affected by ultrasonic waves. However, a conventional ultrasonic diagnostic apparatus requires an operator to observe and check related indications by frequently monitoring various kinds of indication values, images, and the like, which are displayed on a display device of the apparatus while carrying out ultrasonic diagnosis operation. As a result, diagnosis operation can be inconvenient to carry out, and a disadvantageous environment providing a possibility of adversely affecting an unborn baby or the like can be incidentally maintained for a long time due to an operator mistake.

### SUMMARY

To improve over the art and address one or more of the needs outlined above, the present invention provides an ultrasonic diagnostic which, during ultrasonic diagnosis, detects an ultrasonic influence factor that can affect a target body, and gives a warning to a user based on the detected results, thereby performing ultrasonic diagnosis safe for the body.

In accordance with one aspect, the present invention provides an ultrasonic diagnostic apparatus which transmits ultrasonic signals to an object and receives reflected ultrasonic signals from the object to create ultrasonic images. The apparatus includes: a detection unit that detects an ultrasonic influence factor indicative of the influence of ultrasonic waves upon biological tissues; a warning unit that warns a user; and a controller that receives the ultrasonic influence factor from the detection unit and activates the warning unit if the ultrasonic influence factor exceeds a predetermined threshold value.

The ultrasonic influence factor may include at least one of a thermal index (TI), a mechanical index (MI), and a spatial peak temporal average intensity (SPTA).

The warning unit may be an alarm device that generates warning sound or a vibrator that generates vibration.

The warning unit may be mounted on a probe of the ultrasonic diagnostic apparatus.

### BRIEF DESCRIPTION OF THE DRAWINGS

The figures depict one or more implementations in accord with the present teachings, by way of example only, not by way of limitation. In the figures, like reference numerals refer to the same or similar elements.

Fig. 1 is a block diagram of an ultrasonic diagnostic apparatus according to an exemplary embodiment of the present invention;

Fig. 2 is a flowchart explaining operation of the ultrasonic diagnostic apparatus according to the exemplary embodiment; and

Fig. 3 is a view of a probe and a warning unit provided to the ultrasonic diagnostic apparatus according to the exemplary embodiment.

### DETAILED DESCRIPTION

In the following detailed description, numerous specific details are set forth by way of example in order to provide a thorough understanding of the relevant teachings. However, the scope of the present invention is not limited to the specific details.

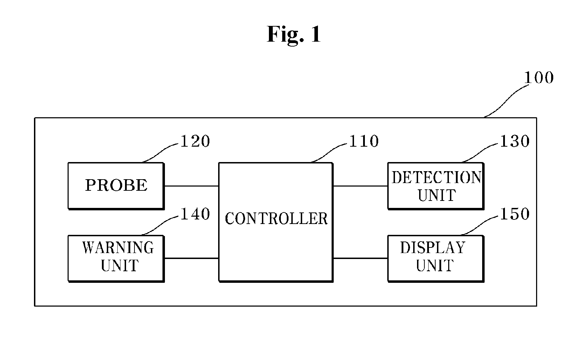

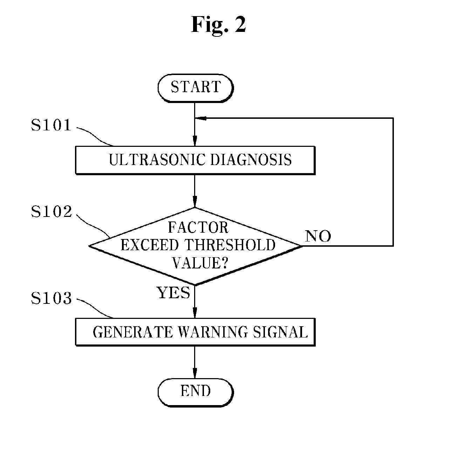

Fig. 1 is a block diagram of an ultrasonic diagnostic apparatus according to an exemplary embodiment of the present invention, and Fig. 2 is a flowchart explaining operation of the ultrasonic diagnostic apparatus according to the exemplary embodiment.

Referring to Fig. 1, an ultrasonic diagnostic apparatus 100 according to an exemplary embodiment transmits ultrasonic signals to an object and receives reflected ultrasonic signals from the object to create ultrasonic images. The apparatus may include a detection unit 130 for detecting an ultrasonic influence factor indicative of influences of ultrasonic waves upon biological tissues, a warning unit 140 warning a user, and a controller 110 receiving the ultrasonic influence factor from the detection unit 130 and activating the warning unit 140 if the ultrasonic influence factor exceeds a predetermined threshold value.

Operation of the ultrasonic diagnostic apparatus 100 according to the embodiment will be described in detail with reference to Figs. 1 to 3.

First, an operator or diagnostician performs ultrasonic diagnosis on an object using the ultrasonic diagnostic apparatus 100 in S101. Specifically, the ultrasonic diagnostic apparatus transmits ultrasonic signals to an object, receives reflected ultrasonic signals from the object, creates ultrasonic images based on the received signals, and outputs the ultrasonic images to a display unit 150.

The detection unit 130 detects the ultrasonic influence factors indicative of influences of ultrasonic waves upon biological tissues and offers the ultrasonic influence factors to the controller 110, so that the display unit 150 displays various kinds of ultrasonic influence factors indicative of the influences upon biological tissues, as well as ultrasonic images.

The ultrasonic influence factor means a variety of indexes or factors that are expressed as indexes or factors indicative of influences of ultrasonic waves upon various parts of the body including soft tissues, bones, and the like, during ultrasonic diagnosis. If the ultrasonic influence factor is out of a proper range, this means that the body can be adversely affected by ultrasonic waves generated from the ultrasonic diagnostic apparatus during ultrasonic diagnosis.

Examples of ultrasonic influence factor include a thermal index (TI) that indicates degrees of ultrasonic waves converted into heat when absorbed into soft tissue or bone of the body, and a mechanical index (MI) that indicates a physical influence of ultrasonic waves upon soft tissues or bones of the body. In addition, the exemplary ultrasonic influence factor may include spatial peak temporal average intensity (SPTA) that indicates the maximum temporal average intensity created in ultrasonic waves. Here, since increase in the SPTA may result in tissue damage due to interference of ultrasonic waves, SPTA is an important ultrasonic influence factor. Obviously, the ultrasonic influence factor is not limited to the TI, MI, and SPTA and any other factors may also be used so long as they can indicate the influence of ultrasonic waves upon the body as a numeric value.

Next, the controller 110 determines whether the ultrasonic influence factor provided from the detection unit 130 exceeds a predetermined threshold value stored in a system. Here, if the factor does not exceed the threshold value, it means normal ultrasonic diagnosis operation, so that the controller controls the ultrasonic diagnostic apparatus 100 to continue to carry out the diagnostic operation.

However, if the factor exceeds the threshold value, the controller 110 controls the warning unit 140 to warn an operator of this situation. Here, the warning unit 140 may be an alarm device that generates warning sound or a vibrator that generates vibration. The warning sound or vibration form the warning unit 140 can warn the user can temporarily stop the ultrasonic diagnosis operation, thereby preventing the ultrasonic waves from doing harm to the body upon ultrasonic diagnosis.

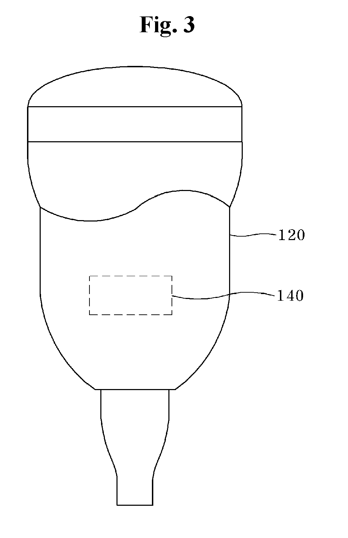

Referring to Fig. 3, the warning unit 140 is mounted on a probe 120 so that an operator of the ultrasonic diagnosis apparatus can easily be aware of the warning.

As set forth above, the ultrasonic diagnostic apparatus according to the embodiment of the present invention detects a variety of ultrasonic influence factors that can affect the body during ultrasonic diagnosis, and determines whether the influence factors are within a proper range, and if the ultrasonic influence factors are out of the proper range, the apparatus warns the user of the situation, thereby enabling ultrasonic diagnosis that is safe for and harmless to the body.

While the foregoing has described what are considered to be the best mode and/or other examples, it is understood that various modifications may be made therein and that the subject matter disclosed herein may be implemented in various forms and examples, and that the teachings may be applied in numerous applications, only some of which have been described herein. It is intended by the following claims to claim any and all applications, modifications and variations that fall within the true scope of the present teachings.

## Claims

1. An ultrasonic diagnostic apparatus which transmits ultrasonic signals to an object and receives reflected ultrasonic signals from the object to create ultrasonic images, **characterized by** comprising:
a detection unit (130) that detects an ultrasonic influence factor indicative of the influence of ultrasonic waves upon biological tissues;
a warning unit (140) that warns a user; and
a controller (110) that receives the ultrasonic influence factor from the detection unit (130) and activates the warning unit if the ultrasonic influence factor exceeds a predetermined threshold value.

2. The apparatus of claim 1, **characterized in that** the ultrasonic influence factor includes at least one of a thermal index (TI), a mechanical index (MI), and a spatial peak temporal average intensity (SPTA).

3. The apparatus of claim 1, **characterized in that** the warning unit (140) is an alarm device that generates warning sound or a vibrator that generates vibration.

4. The apparatus of claim 3, **characterized in that** the warning unit (140) is mounted on a probe (120) of the apparatus.
